# EUROPÄISCHE PATENTANMELDUNG

(11) **EP 4 548 883 A1**
(43) Veröffentlichungstag der Anmeldung: **07.05.2025**
(21) Anmeldenummer: 24020325.7
(22) Anmeldetag: 01.11.2024
(51) Int. Cl.: A61F 2/78

(54) **ENDO-EXO-PROTHESE FÜR GLIEDMASSEN**

(30) Priorität: 02.11.2023 DE 102023130257
(71) Anmelder: Streilau, Helena, 23992 Neukloster (DE)
(72) Erfinder: Streilau, Helena, 23992 Neukloster (DE)
(74) Vertreter: Völger, Karl Wolfgang

(57) **Zusammenfassung**

Die vorliegende Erfindung bezieht sich auf eine Endo-Exo-Prothese (1) für Gliedmaßen umfassend einen länglichen Prothesenkörper (101) mit einem proximalen Ende (103) und einem distalen Ende (105),
wobei das proximale Ende (103) zum teilweisen Einsetzen in einen Knochen (K) ausgelegt ist und
wobei am distalen Ende (105) eine mechanische Aufnahme (107) für weitere Elemente vorgesehen ist.

Ferne wird ein Verfahren zum Anbringen einer Endo-Exo-Prothese (1) offenbart.

## Beschreibung

Die vorliegende Erfindung betrifft eine Endo-Exo-Prothese für Gliedmaßen, insbesondere für die unteren Gliedmaßen, hier insbesondere für die Tibia, sowie ein Verfahren zu deren Anbringen.

Prothesen werden nach Amputation von Gliedmaßen genutzt, um die Funktion des amputierten Körperteils ganz oder teilweise zu ersetzen. Dabei werden verschiedene Arten von Prothesen unterschieden. Endoprothesen sind vollständig von Körpergewebe umschlossen. Ein klassisches Beispiel ist das künstliche Kniegelenk. Exoprothesen wiederum befinden sich außerhalb vom Körper. Hierbei handelt es sich um die klassischen Arm-, Bein und Handprothesen. Offene Implantate, wie Zahnimplantate, sind im Körper verankert, ragen aber auch aus diesem heraus. Mit der Endo-Exo-Prothese wird hingegen mehr als ein Körperteil wie der Zahn ersetzt. Dabei wird auch die Mobilität des Patienten erhalten.

Aus dem Stand der Technik sind seit vielen Jahren die unterschiedlichsten Prothesen bekannt. Klassische Unterschenkelprothesen werden beispielsweise in DE 38 08 994 C2 beschrieben, wobei diese Prothese am Liner (Hülsenteil) des Stumpfes angebracht wird und durch Verwendung von CFK federnd ausgestaltet ist.

DE 10 2005 034 388 B4 offenbart einen Kupplungsmechanismus der Prothese mit dem Liner, hier liegt eine gute Beschreibung beider üblicher Verfahren Unterdruck / mechanische Kupplung) vor. Der Kupplungsmechanismus dient zur Befestigung der fehlenden, d.h. amputierten, Körperteile.

In DE 43 38 746 C2 wird eine Art zweiteiliger Knochennagel angegeben, um diesen einerseits in die Tibia einzuführen und andererseits mit einem eigenen "Nagel" auch in der Fibula zu verankern.

DE 693 24 930 T2 betrifft die Auswechselbarkeit der an der Grundprothese angebauten Teile, hier eine zylindrische Rohrstütze mit der Möglichkeit zur Montage verschiedener "Füße".

Verschiedene Schriften geben zudem überwundene Nachteile klassischer Prothesen an, so beispielsweise DE 94 19 211 U1 mit den Nachteilen einer klassischen Unterschenkelprothese wie Belüftung des Stumpfes und selbstständiger Anpassung. DE 10 2017 120 257 A1 beschreibt einen alternativen Adapter, um möglichst schnell zwischen verschiedenen Anwendungen wechseln zu können.

Alle vorgestellten Lösungen stellen klassische Prothesen dar, die über einen Liner am Körper befestigt werden, oder im Falle des Marknagels der Stabilisierung einzelner Teile des Körpers dienen. Liner müssen individuell für den jeweiligen Stumpf gefertigt und angepasst oder verändert werden, wenn sich die körperliche Konstitution des Patienten ändert.

Der vorliegenden Erfindung liegt daher die Aufgabe zu Grunde, die Nachteile des Standes der Technik zu überwinden und eine Prothese bereitzustellen, die durch ihre Ausführung als Endo-Exo-Prothese keine Hautirritationen induziert und die dem Nutzer die Möglichkeit bietet für, verschiedene Anwendungen verschiedene Funktionsteile zu verwenden, sowie ein OP-Verfahren anzugeben, mit dem diese Endo-Exo-Prothese im Körper zu verankern ist.

Diese Aufgabe wird in der vorliegenden Erfindung in einem ersten Aspekt durch eine Endo-Exo-Prothese (1) für Gliedmaßen, vorzugsweise Unterschenkel, insbesondere der Tibia, gelöst, umfassend einen länglichen Prothesenkörper (101) mit einem proximalen Ende (103) und einem distalen Ende (105),
wobei das proximale Ende (103) zum teilweisen Einsetzen in einen Knochen (K) ausgelegt ist und
wobei am distalen Ende (105) eine mechanische Aufnahme (107) für weitere Elemente vorgesehen ist.

Die Begriffe "proximal" (dem Körper zugewandt) und "distal" (dem Körper abgewandt) werden gemäß der in der Medizin bekannten und üblichen Verwendung angegeben.

Bei den Gliedmaßen handelt es sich insbesondere um die unteren Gliedmaßen, so dass es sich bei dem Knochen (K) bevorzugt um einen Röhrenknochen handelt.

Die mechanische Aufnahme (107) kann beispielsweise ein Gewinde, eine Art Bajonettverschluss oder dergleichen umfassen.

Die vorliegende Erfindung hat den Vorteil, dass mit der Endo-Exo-Prothese (1) eine stabile und fest mit dem Körper verbundene Grundlage geschaffen wird, an der sich verschiedene Einzelprothesen anbringen lassen. Damit überträgt die vorliegende Erfindung das aus der Zahnheilkunde in anderer Weise bekannte Prinzip der offenen Implantate auf den Bewegungsapparat, insbesondere die unteren Extremitäten. Während im Mundraum nahezu optimale Bedingungen für eine offene Einheilung von Zahnimplantaten herrscht und die auftretenden Kräfte gut beherrschbar sind, war es für die vorliegenden Erfindung eine Herausforderung, die im Bewegungsapparat auftretenden Kräfte, die neben Druck auch Zug in Torsion umfassen, zu beherrschen.

Bei der erfindungsgemäßen Endo-Exo-Prothese (1) handelt es sich bei den Gliedmaßen um den Unterschenkel, insbesondere um die Tibia.

Bei diesen Gliedmaßen sind die Vorteile der erfindungsgemäßen Endo-Exo-Prothese (1) besonders groß, wie nachstehend noch herausgearbeitet wird.

Es hat sich für das problemlose Einführen der erfindungsgemäßen Endo-Exo-Prothese (1) in den Knochen (K) als vorteilhaft herausgestellt, wenn das proximale Ende (103) zur Einführung in den Knochen (K) abgerundet ausgebildet ist.

Eine Ausführungsform der erfindungsgemäßen Endo-Exo-Prothese (1) sieht vor, dass unterhalb des proximalen Endes (103) eine radial angeordnete ringförmige, offenporige Struktur (109) vorgesehen ist, um das distale Ende des Knochens (K) mit direktem Kontakt aufzunehmen.

Mit dem Vorsehen der radial angeordneten ringförmigen, offenporigen Struktur (109) wird ein besserer Halt der erfindungsgemäßen Endo-Exo-Prothese (1) am / im Knochen (K) erreicht. Zunächst wird verhindert, dass bei Belastung der erfindungsgemäßen Endo-Exo-Prothese (1) der längliche Prothesenkörper (101) tiefer in den Knochen (K) eindringt, als sinnvoll und vorgesehen ist. Da ferner der Knochen (K) nach dem Absetzen eines Teils der Gliedmaße an seinem distalen Ende freiliegt, ist es vorteilhaft, wenn dieser nach dem Einsetzen der erfindungsgemäßen Endo-Exo-Prothese (1) auf dem offenporigen Teil aufliegt und diesen mit dem weiteren Knochenwachstum mit der Zeit durchdringen kann.

In einer Weiterbildung der erfindungsgemäßen Endo-Exo-Prothese (1) ist mindestens eine Lasche (111) am äußeren Umfang der ringförmigen, offenporigen Struktur (109) zur kraftschlüssigen Fixierung der Endo-Exo-Prothese (1) am Knochen (K) vorgesehen. Mit dieser mindestens einen Lasche (111) - auch als "LC-Platte" (locking compression) bezeichnet - kann die Endo-Exo-Prothese (1) bspw. durch Verschrauben befestigt werden.

Es hat sich für die sichere Befestigung der erfindungsgemäßen Endo-Exo-Prothese (1) als vorteilhaft herausgestellt, wenn diese ferner ein scheibenförmiges Element (113), das am äußeren Rand der ringförmigen, offenporigen Struktur (109) vorgesehen ist, wobei das scheibenförmige Element (113) an seinem Rand zirkulär perforiert und mit mehreren Öffnungen versehen ist.

Das scheibenförmige Element (113) kann nach dem Einbringen der erfindungsgemäßen Endo-Exo-Prothese (1) in den Knochen (K) umgebogen werden, so dass es am Knochenstumpf anliegt. Die Perforation erleichtert das Umbiegen, während die Löcher zum Festschrauben dienen können.

Um die Muskelschichten der Gliedmaße sicher zu fixieren, sieht eine Ausführungsform vor, dass die erfindungsgemäße Endo-Exo-Prothese (1) ferner mindestens eine radial durchgehende Bohrung (115) unterhalb der ringförmigen, offenporigen Struktur (109) umfasst. Durch diese Bohrung (115) können die Muskelenden gezogen werden, um diese zu fixieren. Die durchgehende Bohrung (115) hat insbesondere einen ovalen Querschnitt.

Die Muskulatur kann auf diese Weise gespannt werden. Dies ist besonders vorteilhaft, weil sich nur eine vorgespannte Muskulatur kontrahieren kann. Bei der Unterschenkelamputation, also der Durchtrennung der Tibia, werden zwar vorrangig jene Muskeln durchgetrennt, die primär für die Bewegung des oberen Sprunggelenkes, des Fußes und der Zehen zuständig sind, welche offenbar nach der Unterschenkelamputation nicht mehr vorhanden sind, dennoch tragen eben jene Muskeln zur Stabilisierung des Kniegelenks bei.

Die Befestigung und Vorspannung des Muskels ist dabei besonders vorteilhaft, weil so eine vorgespannte Muskulatur durch, z. B. ein EMS-Training (Elektromyostimulation) aufrecht erhalten werden kann und es nicht zu stark zu einer Muskelathrophie kommt.

Mit dem EMS-Training kann aber frühestens nach abgeschlossener Narbenbildung, was beim Muskelgewebe in ca. 12 Wochen nach der Operation der Fall ist, begonnen werden. Zu diesem Zeitpunkt wird noch genügend Muskulatur vorhanden sein, um es dann mittels Elektrostimulation aufzubauen. Ein Muskel, der nur am proximalen Ende im Körper vorhanden ist, kann sich kaum kontaktieren, es kommt zu einer fettigen Degeneration, auf keinem Fall kann so ein Muskel zur Stabilität des Kniegelenkes beitragen, was durch die Befestigung des Muskels verhindert wird.

In einer Weiterbildung dieser Ausführungsform sind zwei durchgehende Bohrungen (115a, 115b) vorgesehen, wobei die proximal angeordnete durchgehende Bohrung (115a) die tieferen Muskelschichten aufnimmt und die distal angeordnete durchgehende Bohrung (115b) für die oberflächlichen Muskelschichten vorgesehen ist. Dadurch wird der Ansatz dieser Muskelschichten neu geschaffen, so dass die Muskelgruppen unter einer gewissen Spannung stehen, um sich weiterhin kontrahieren zu können.

Die erfindungsgemäße Endo-Exo-Prothese (1) ist gemäß einer Weiterbildung im Wesentlichen aus Stahl, insbesondere sog. "Chirurgenstahl", gefertigt, wobei alle Flächen, welche mit organischem Material direkten Kontakt haben, mit Gold oder Silber beschichtet sind. Dies erhöht die Biokompatibilität und verringert das Risiko von Abstoßungsreaktionen. Weiterhin hat das Silber erwiesenermaßen bakteriostatische bzw. bakterizide Wirkung, das Gold ist besonders allergenarm und gut gewebeverträglich.

Eine spezielle Ausführungsform der erfindungsgemäßen Endo-Exo-Prothese (1) sieht vor, dass das distale Ende (105) sich außerhalb der Gliedmaße befindet und die mechanische Aufnahme (107) dazu ausgeführt ist, Funktionsteile (3) mit der Endo-Exo-Prothese (1) kraftschlüssig und/oder formschlüssig lösbar zu verbinden. Die mechanische Aufnahme (107) ist dabei bevorzugt aus Stahl gefertigt.

Diese Ausführungsform bietet den Vorteil, dass unterschiedliche Arten von Einzelprothesen für verschiedene Anwendungsfälle befestigt werden können.

Insbesondere kann das Funktionsteil (3) für die erfindungsgemäße Endo-Exo-Prothese (1) kugelförmig (3a) oder als stilisierter Fuß (3b) ausgebildet sein.

Während ein kugelförmiges Funktionsteil (3a) beispielsweise zum Schutz bzw. der Abdeckung der mechanischen Aufnahme (107) dienen kann, ist ein stilisierter Fuß (3b) für das gewöhnliche Laufen konstruiert. Weitere Funktionsteile (3) sind denkbar, z.B. spezielle Ausführungen für verschiedene Sportarten.

Die vorstehend genannte Aufgabe wird in einem zweien Aspekt der vorliegenden Erfindung durch ein Verfahren zum Anbringen einer erfindungsgemäßen Endo-Exo-Prothese (1) an einer Gliedmaße gelöst, umfassend die Schritte
a) Absetzen eines Knochens (K) einer Gliedmaße,
b) Anbringen der Endo-Exo-Prothese (1), umfassend die Unterschritte
c) Einsetzen des proximalen Endes (103) der Endo-Exo-Prothese (1) in den Knochen (K), insbesondere in die Knochenmarkhöhle, optional bis zum direkten Kontakt mit der ringförmigen, offenporigen Struktur (109),
d) optional Festschrauben der zumindest einen Lasche (111) am Knochen (K),
e) optional Umbiegen des radial angeordneten scheibenförmigen Elements (113) um das distale Ende des Knochens (K) und optional Festschrauben am Knochen (K),
f) optional Präparation von Muskeln der Gliedmaße und Befestigung der Muskelstränge in der mindestens einen radial durchgehenden Bohrung (115).

Das erfindungsgemäße Verfahren bietet grundsätzlich die gleichen Vorteile wie die erfindungsgemäße Endo-Exo-Prothese (1), die damit befestigt wird. Das Verfahren stellt kein "Verfahren zur chirurgischen oder therapeutischen Behandlung des menschlichen oder tierischen Körpers" im Sinne des Patentgesetzes dar, sondern ein rein mechanisches Verfahren, bei dem die Erhaltung des Lebens oder der Gesundheit des Körpers nicht von entscheidender Bedeutung ist. Das erfindungsgemäße Verfahren führt in vorteilhafter Weise dazu, die erfindungsgemäße Endo-Exo-Prothese (1) stabil und fest mit dem Körper zu verbinden und damit eine Grundlage zu schaffen, an der sich verschiedene Einzelprothesen anbringen lassen.

Bekanntermaßen werden Gelenke des Körpers durch die Muskulatur stabilisiert. Bei herkömmlichen Operationsmethoden zur Amputation wird die Unterschenkenmuskulatur i.d.R. abgeschnitten und evtl. mit dem Periost des Knochenstumpfs vernäht. Diese Verbindung wird oft durch die Scherkräfte während der Mobilisation in der herkömmlichen prothetischen Versorgung gerissen, so dass im es zu deren Degeneration und zu einem sog. störenden Weichteilüberschuss kommt.

Durch den erfindungsgemäßen Schritt f) wird eine solche Degeneration vermieden, da fast die ursprüngliche Lage der Muskulatur beibehalten wird. Die solchermaßen vorgespannte Muskulatur, die zudem nicht denerviert ist, behält Ihre Fähigkeit zur Kontraktion, wodurch das Kniegelenk weiterhin stabilisiert wird. Weiterhin ist auch das aktive Beugen des Kniegelenks möglich.

Weitere Ziele, Merkmale, Vorteile und Anwendungsmöglichkeiten ergeben sich aus der nachfolgenden Beschreibung von, die Erfindung nicht einschränkenden Ausführungsbeispielen, anhand der Figur. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1:: eine schematische Darstellung der erfindungsgemäßen Endo-Exo-Prothese 1 nach einer Ausführungsform der Erfindung, Fließschema des erfindungsgemäßen Verfahrens,
- Fig. 2:: eine schematische Detaildarstellung eines Teils der in Fig. 1 gezeigten erfindungsgemäßen Endo-Exo-Prothese 1,
- Fig. 3: eine schematische Darstellung eines kugelförmigen Funktionsteils 3a,
- Fig. 4: eine schematische Darstellung eines fußförmigen Funktionsteils 3a und
- Fig. 5: eine Kompressionsstrumpfhose der Klasse II zur Nachsorgebehandlung.

In den Figuren werden alle gleichen Teile mit den gleichen Bezugszeichen benannt, aus Gründen der Übersichtlichkeit sind aber nicht unbedingt in allen Darstellungen alle Bezugszeichen eingefügt.

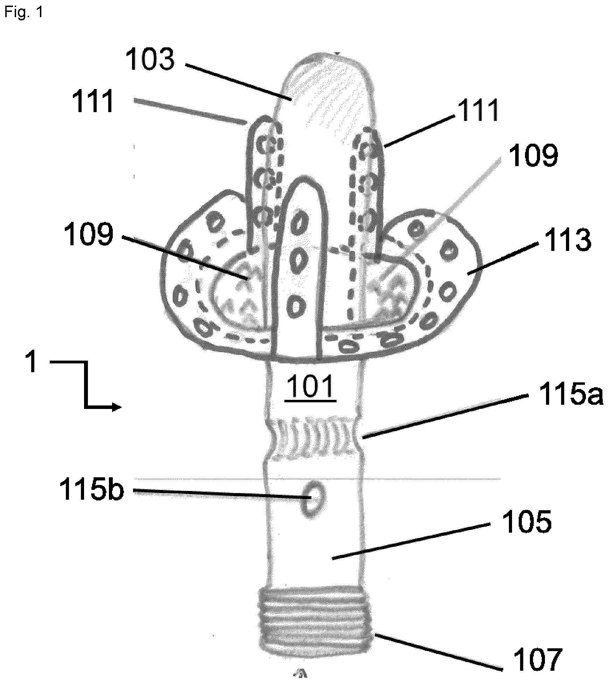

Figur 1 zeigt schematisch die erfindungsgemäße Endo-Exo-Prothese 1 in einer bevorzugten Ausführung. Zentrales Element ist der längliche Prothesenkörper 101 mit dem proximalen Ende 103 zum zumindest teilweisen Einsetzen in einen Knochen K ausgelegt und dem distalen Ende 105 mit mechanischer Aufnahme 107. In Richtung des distalen Endes 105 weist der längliche Prothesenkörper 101 zwei radial durchgehende Bohrungen 115a, 115b auf, die versetzt zueinander angeordnet sind. Der Querschnitt der durchgehenden Bohrungen 115a, 115b ist vorzugsweise oval. Die mechanische Aufnahme 107 ist in dieser Ausführungsform vorzugsweise als Außengewinde ausgebildet.

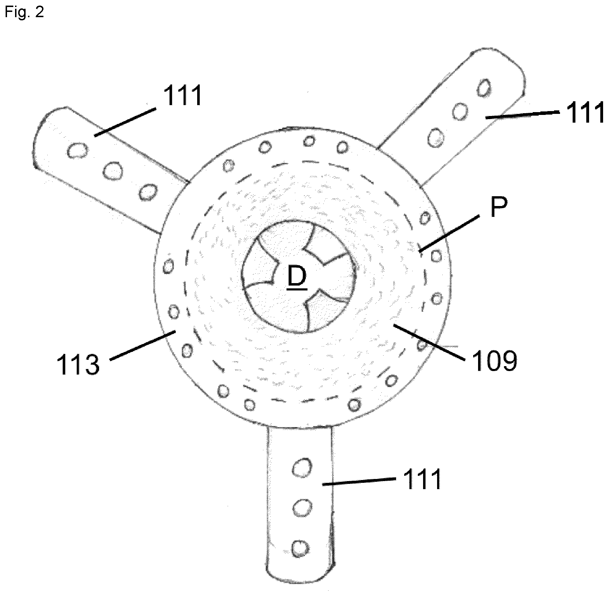

Um den länglichen Prothesenkörper 101 herum ist die sog. Prothesenbasis angeordnet, die in Figur 2 noch einmal im Detail dargestellt wird. Diese sog. Prothesenbasis weist mittig einen Durchgang D auf, durch welchen der längliche Prothesenkörper 101 durchtritt, bis er die richtige Position erreicht. In dem Durchgang D können Laschen (hier ohne Bezugszeichen) vorgesehen sind, die sich beim Durchführen an den länglichen Prothesenkörper 101 anlegen und anschließend befestigt sind, z. B. verschweißt sind, bspw. geschweißt werden.

Um den Durchgang D und damit direkt an den länglichen Prothesenkörper 101 angrenzend ist radial eine ringförmige, offenporige Struktur 109 angeordnet, die mit dem distalen Ende des Knochens K in direkten Kontakt gebracht werden kann. Um die ringförmige, offenporige Struktur 109 ist dann in dieser Ausführungsform ein scheibenförmiges Element 113 über zirkuläre Perforationen P vorgesehen, das mit mehreren Öffnungen versehen ist. Die Perforationen P erleichtert das Umbiegen des scheibenförmigen Elements 113, das über die Öffnungen mit dem Knochen K bspw. verschraubt werden kann.

Am äußeren Rand des scheibenförmigen Elements 113 sind zudem vorzugsweise Laschen 111 vorgesehen, welche als sog. LC Platten ausgeführt sein können. Auch diese werden über die darin vorhandenen Öffnungen mit dem Knochen K verbunden.

In Figur 1 wird die erfindungsgemäße Endo-Exo-Prothese 1 in einem grundsätzlich montierten Zustand, allerdings zur besseren Sichtbarkeit ohne Knochen K dargestellt.

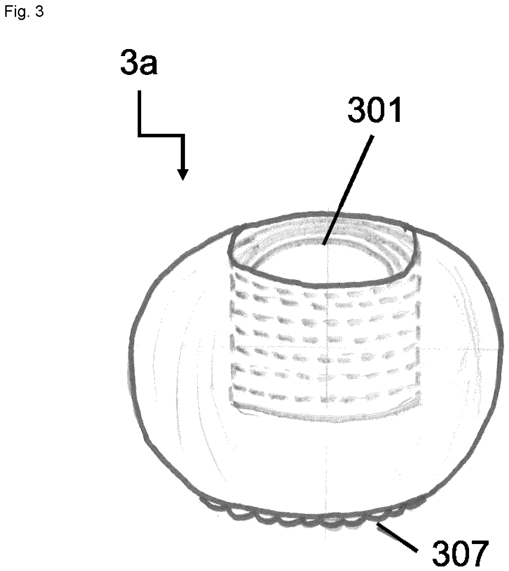

In Figur 3 wird schematische das Funktionsteil 3 in Kugelform 3a gezeigt. Das Funktionsteil 3 weist ein Innengewinde 301 auf, dass passend zum Gewinde der mechanische Aufnahme 107 ausgelegt ist. Auf der Unterseite kann das Funktionsteil 3 eine Lauffläche, bspw. aus profiliertem Gummi, aufweisen.

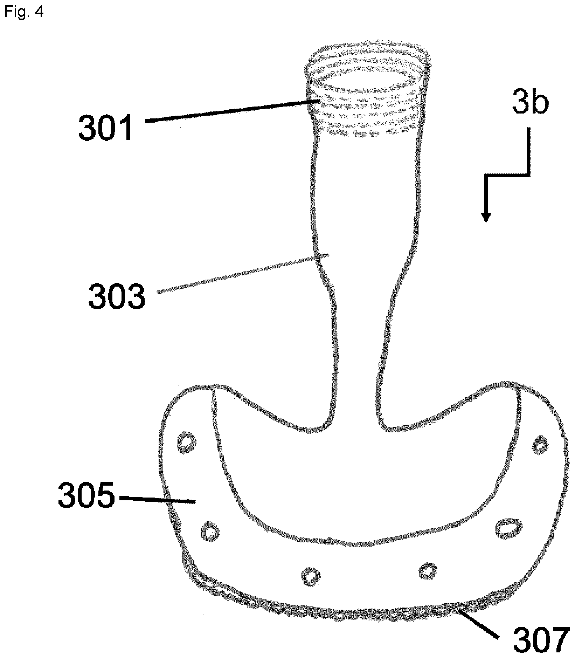

In Figur 4 wird schematische das Funktionsteil 3 in Fußform 3b gezeigt. Das Funktionsteil 3 weist wieder ein Innengewinde 301 passend zum Gewinde der mechanische Aufnahme 107 auf. Im Mittelteil ist ein der Körpergröße des Trägers angepasster Schaft 303 vorgesehen, der in den halbrund, bzw. mit Rundung an der Unterseite ausgebildeten Fußteil 305 übergeht. Auf der Unterseite kann das Funktionsteil 3 hier ebenfalls eine Lauffläche 307, bspw. aus profiliertem Gummi, aufweisen.

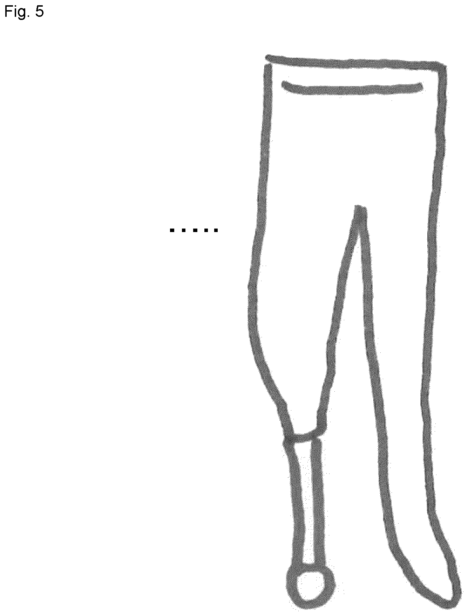

Figur 5 zeigt eine Kompressionsstrumpfhose der Klasse II zur postoperativen Nachsorgebehandlung nach der durchgeführten Amputation und Implantation der erfindungsgemäßen Endo-Exo-Prothese 1, hier am Beispiel einer Unterschenkelamputation rechts. Diese Strumpfhose sollte nach Maß angefertigt werden und ab dem 7. postoperativen Tag getragen werden. Bis dahin soll die Strumpfformung durch die Wickelung des Stumpfs erfolgen. Die Strumpfhose soll zur weiteren Stumpfformung und Thromboseprophylaxe dienen. Diese Strumpfhose sollte nur zum Duschen bzw. Ganzkörperpflege oder Verbandswechsel ausgezogen werden. Die Stumpfformung auf diese Weise sollte bis zur 12. postoperativen Woche fortgeführt werden. Ab der 6. postoperativen Woche sollte die Passgenauigkeit der Strumpfhose überprüft, bzw. eine neue Strumpfhose angefertigt werden.

Nachstehend wird die vorliegende Erfindung in einer bevorzugten Ausführungsform beschrieben, ohne diese darauf zu beschränken.

Das proximale, abgerundete Ende 103 der erfindungsgemäßen Endo-Exo-Prothese 1 wird in den Knochen K, konkret in den Markkanal eingeführt. Die Fibula wird schräg von proximal lateral nach medial distal amputiert und verbleibt dann so im Stumpf. Da die Muskulatur eigentlich an den distalen Teil der Prothese adoptiert wird, verbleibt die amputierte Fibula so im Stumpf. Sie würde nicht weiter bei der Mobilisation stören. Wenn man die Fibula so im Amputationsverbund belässt, geht man auch nicht die Gefahr, N. fibularis longus zu verletzen.

Die Schnittfläche des Knochens K liegt auf der radial angeordneten ringförmigen, offenporigen Struktur 109, vorzugsweise aus porösem Titan, auf, welche mit dem scheibenförmigen Element 113 um den Knochen K herum gebogen wird, und dem Knochen K die Möglichkeit bietet, mit diesem zu verwachsen.

Titan hat diesbezüglich den Vorteil, dass es vom Körper sehr gut angenommen wird und sehr selten zu Abstoßungsreaktionen beobachtet werden können. Zusätzlich wird die erfindungsgemäße Endo-Exo-Prothese 1 mit dem Knochen K verschraubt. Bevorzugt werden dabei sogenannte LC Platten verwendet. LC bezeichnet "Locking Compression" Platten, welche sich dadurch auszeichnen, dass der Kopf der zur Befestigung dienenden Schraube fest in der LC Platte verankert wird. Diese LC Platten sind (indirekt) mit der ringförmigen, offenporigen Struktur 109 aus Titan verbunden und bereits vorzugsweise, wenn jene um den Knochenstumpf gebogen werden sollen, in Richtung des Knochens K nach proximal vorgebogen, so dass nur noch ggf. eine geringfügige Anpassung während des Anschraubens erfolgen muss. In der dargestellten bevorzugten Ausführungsform werden drei über den Umfang verteilte LC Platten verwendet. Denkbar sind auch Ausführungen mit mehr oder weniger LC Platten, mindestens jedoch einer LC Platte. Distal zur ringförmigen, offenporigen Struktur 109 befinden sich zwei um vorzugsweise 90° radial durchgehende Bohrungen 115 in dem länglichen Prothesenkörper 101, um die Muskulatur an der erfindungsgemäßen Endo-Exo-Prothese 1 zu befestigen.

Ein weiterer Aspekt der erfindungsgemäßen Endo-Exo-Prothese 1 ist, dass diese vollständig aus Chirurgenstahl (AISI Bezeichnung 316L) gefertigt ist, sowie an allen Flächen, welche mit organischem Material direkten Kontakt haben, mit Gold oder Silber beschichtet ist, um eine bessere Materialverträglichkeit zu gewährleisten.

Das distale Ende der erfindungsgemäßen Endo-Exo-Prothese 1 weist eine mechanische Aufnahme 107, bevorzugt ein Gewinde, auf, welches aus dem Körper des Trägers/Patienten herausragt. Auf dieses Gewinde 107 wird nach dem Anlegen des Verbandes eine Kugel oder ein Konus geschraubt, mit dem ein Hängenbleiben z.B. an der Bettwäsche oder in der Kleidung verhindert werden soll.

Das Gewinde 107 dient zur kraftschlüssigen Aufnahme aller denkbaren Funktionsteile 3, bspw. einer Kugel 3a, eines Gehfußes 3b, eines Schwimmfußes oder weiterer, nicht näher spezifizierter Teile. Jegliche Funktionsteile 3 weisen ein Innengewinde 301 auf. Besonders vorteilhaft ist es beispielsweise eine Kugel 3a zu verwenden, welche die erfindungsgemäße Endo-Exo-Prothese 1 beispielsweise vor umgebenden Textilien schützt sowie kurzes Stehen und/oder kurze Laufstrecken ermöglicht.

Die Wundversorgung und alle konkreten Schritte bei der Operation zum Einsetzen der Prothese sollen im nächsten Aspekt der Erfindung beschrieben werden.

Dem auf dem Rücken liegenden Patienten wird, außer bei einer Kontraindikation, eine Oberschenkel Blutsperre gelegt und die Haut nicht weit zurück abpräpariert.

Zur Präparation der Haut wird diese nicht weit zurück abpräpariert. Dabei soll darauf geachtet werden, dass die Hautlappen großzügig bemessen sind und die Blutgefäße, die durch die Faszie zur Haut ziehen, dadurch geschont werden. Das gefäßtragende subkutane Fettgewebe verbleibt an der Haut. Die Muskeln werden schräg zentral konvergierend, möglichst nah an der Ansatzsehne, aber im Gesunden, vitalen Gewebe durchtrennt. Anschließend werden die kräftigen Blutgefäße legiert (abgebunden) und dann durchtrennt.

Die Nerven werden sanft hervorgezogen und mit einer scharfen Klinge gekürzt, wodurch sie in die Muskulatur zurück schlüpfen.

Das Periost wird etwa sechs bis zehn Zentimeter distal des Wundrandes quer durchtrennt und drei bis vier Zentimeter schlauchförmig zurück präpariert bis zur Osteotomiestelle. Nach der Durchtrennung des Knochens K (Osteotomie) wird der Knochenrand abgefeilt und die Tibiaspitze wird rund gefeilt, so dass keine Spitze hervorsteht.

Der Patient ist nun für das Einsetzen der erfindungsgemäßen Endo-Exo-Prothese 1 bereit, wobei das abgerundete proximale Ende 103 der erfindungsgemäßen Endo-Exo-Prothese 1 in die Knochenmarkhöhle geschoben wird, bis die ringförmige, offenporige Struktur 109 am Knochen K anliegt.

Anschließend erfolgt das Anbringen der Schrauben an die Laschen 111, hier als LC Platten, darunter wird erst das Periost abpräpariert, so dass die LC-Platten 111 nur auf den Knochen angebracht werden, das scheibenförmige Element wird an den Perforationsstellen P um den Knochenrand umgebogen und ggf. mit einigen Schrauben am Knochen K befestigt werden, so dass die Prothese fest am Knochen K sitzt. Nun wird der abpräpariertere Periostschlauch hervorgezogen, über die ringförmige, offenporige Struktur 109 gezogen und darunter vernäht.

Nun erfolgt die Myoplastie. Die Muskelgruppen werden freipräpariert und die Septen zwischen den Muskeln gespalten. Die einzelnen Muskelgrippen sollen so mobilisiert werden. Wenn mehrere Muskelschischichten vorhanden sind, dann wird die tiefere Muskelschicht kürzer abgetrennt als die oberflächliche Muskelschicht.

Um die Muskulatur an der erfindungsgemäßen Endo-Exo-Prothese 1 zu befestigen, müssen folgende Schritte umgesetzt werden. Die tiefere Muskelschicht wird kürzer abgetrennt als die oberflächliche Muskelschicht. Um die Muskulatur an der erfindungsgemäßen Endo-Exo-Prothese 1 zu befestigen, müssen folgende Schritte umgesetzt werden.

Die tiefere Muskelschicht wird schrägt angeschnitten, von außen distal nach innen proximal und so abgelängt, dass die Muskelschichten von beiden Seiten des Knochens K mit den Fäden durch die proximale, dem Körper nähere radial durchgehende Bohrung 115a in der erfindungsgemäßen Endo-Exo-Prothese 1 verbunden werden können. Die Muskeln sollen unter einer leichten Spannung stehen und können dafür nachgekürzt werden. In einer besonderen Ausführungsform wird zum Zusammenfügen der tiefen Muskelschicht ein doppelt armierter 3x0-PDS Faden und zwei gerade Nadeln verwendet.

In einer besonderen Ausführungsform wird dafür auf einer Seite der so präparierten tiefen Muskelschicht am distalen Ende eine Bunnel-Naht (sog. "Schnürsenkelnaht") gesetzt und die Nadeln werden durch die proximale radial durchgehende Bohrung 115a, die von rechts nach links verläuft, zur Gegenseite gezogen, wo ebenfalls eine Bunnel-Naht gesetzt wird. Das Gleiche wird dann mit der tiefen Muskelschicht der kontralateralen Seite durchgeführt.

Diese so mit einer Bunnel-Naht versorgten Muskelschichten werden außerhalb der radial durchgehenden Bohrung 115a fest zusammengebunden und dann in die radial durchgehende Bohrung 115a zurück geschoben.

Bei der oberflächlichen Muskulatur erfolgt ein analoges Vorgehen, wobei hier zur Befestigung der Muskulatur die distale radial durchgehende Bohrung 115b Bohrung genutzt wird. Die radial durchgehende Bohrung 115b verläuft dabei von vorn bis nach hinten. Sollte beim Patienten nur eine schwache Muskulatur vorhanden sein, wird diese komplett in der proximalen radial durchgehenden Bohrung 115a verankert und die distale radial durchgehende Bohrung 115b verschlossen.

Anschließend erfolgt das Eröffnen der Oberschenkel-Blutsperre und eine sorgfältige Blutstillung.

In einer besonderen Ausfertigungsform ist es auch denkbar, generell nur mit einer radial durchgehenden Bohrung 115 in der erfindungsgemäßen Endo-Exo-Prothese 1 zu arbeiten.

Vor dem Hautverschluss (d.h. Wundverschluss) ist die Lage der erfindungsgemäßen Endo-Exo-Prothese 1 durch eine Röntgenkontrolle zu bestätigen. Die Haut wird anschließend fünf bis sechs Zentimeter unterhalb der distalen radial durchgehenden Bohrung 115b waagerecht gerade abgeschnitten, so dass ein sicher spannungsfreier Hautverschluss um den Prothesenschaft gelingt. Die Haut wird gerafft und mit einer doppelten zirkulären und gegenläufigen Tabaksbeutelnaht vernäht, so dass die Haut gut den Schaft der erfindungsgemäße Endo-Exo-Prothese 1 abdichtet. Vorsicht ist hier vor Hautnekrosen zu nehmen, d.h. die Haut nicht so stark zusammenziehen, dass sie weiß erscheint. Die geraden Nadeln werden seitlich ausgeleitet und die Naht mit mehreren Knoten verschlossen. mit mehreren Knoten verknotet, dann abgeschnitten.

In einer besonderen Weiterbildung kann auf das Legen einer Drainage verzichtet werden, da durch das Ausleiten der Drainage zusätzlich eine Verletzung der Haut besteht. Die Wundflüssigkeit kann aus der OP-Wunde entlang der erfindungsgemäßen Endo-Exo-Prothese 1 ausfließen.

Zum Anlegen des sterilen Verbandes (noch auf dem OP-Tisch) wird das amputierte Bein hochgehalten, die Hautränder mit einer Fettgaze abgedeckt und darauf ein lockerer Wölkchenverband angelegt, welcher mit sterilen Mullbinden abgedeckt wird. Zunächst erfolgt das Anlegen eines sterilen Verbandes vom distalen Ende 105 der erfindungsgemäßen Endo-Exo-Prothese 1 über das Kniegelenk bis zur Mitte des Oberschenkels. In gleicher Richtung erfolgt ein Verband mit einer elastischen Binde in Kreuztechnik um die konische Formung des Stumpfes zu starten. Schließlich erfolgt das Abdecken der sterilen Tücher, auf das distale Ende 107 wird das kugelförmige Funktionsteil 3a geschraubt und er Patient wird vom OP-Tisch umgelagert.

Zur weiteren Wundversorgung wird klassische Nachsorge mit zeitweisem Hochlagern des amputierten Beines, einer Thromboseprophylaxe, Analgetika, Stumpfwickelung, später dann mit Kompressionsstrumpfhose, Rehabilitationsmaßnahmen und Physiotherapie empfohlen.

### Bezugszeichenliste

- 1: Endo-Exo-Prothese
- 101: länglicher Prothesenkörper
- 103: proximales Ende
- 105: distales Ende
- 107: mechanische Aufnahme
- 109: ringförmige, offenporige Struktur
- 111: Lasche (als LC-Platte)
- 113: scheibenförmiges Element
- 115: radial durchgehende Bohrung
- 115a: radial durchgehende Bohrung proximal
- 115b: radial durchgehende Bohrung distal
- 3: Funktionsteil
- 3a: kugelförmiges Funktionsteil
- 3b: stilisierter Fuß
- 301: Innengewinde
- 303: angepasster Schaft
- 305: Fußteil
- 307: Lauffläche

- D: Durchgang
- K: Knochen

## Patentansprüche

1. Endo-Exo-Prothese (1) für Gliedmaßen umfassend einen länglichen Prothesenkörper (101) mit einem proximalen Ende (103) und einem distalen Ende (105),
wobei das proximale Ende (103) zum teilweisen Einsetzen in einen Knochen (K) ausgelegt ist und
wobei am distalen Ende (105) eine mechanische Aufnahme (107) für weitere Elemente vorgesehen ist.

2. Endo-Exo-Prothese (1) nach Anspruch 1, wobei es sich bei den Gliedmaßen um den Unterschenkel handelt, insbesondere um die Tibia.

3. Endo-Exo-Prothese (1) nach Anspruch 1 oder 2, wobei das proximale Ende (103) zur Einführung in den Knochen (K) abgerundet ausgebildet ist.

4. Endo-Exo-Prothese (1) nach einem der Ansprüche 1 bis 3, wobei unterhalb des proximalen Endes (103) eine radial angeordnete ringförmige, offenporige Struktur (109) vorgesehen ist, um das distale Ende des Knochens (K) mit direktem Kontakt aufzunehmen.

5. Endo-Exo-Prothese (1) nach Anspruch 4, wobei mindestens eine Lasche (111) am äußeren Umfang der ringförmigen, offenporigen Struktur (109) zur kraftschlüssigen Fixierung der Endo-Exo-Prothese (1) am Knochen (K) vorgesehen ist.

6. Endo-Exo-Prothese (1) nach einem der Ansprüche 1 bis 5, ferner umfassend ein scheibenförmiges Element (113), das am äußeren Rand der ringförmigen, offenporigen Struktur (109) vorgesehen ist, wobei das scheibenförmige Element (113) an seinem Rand zirkulär perforiert und mit mehreren Öffnungen versehen ist.

7. Endo-Exo-Prothese (1) nach einem der Ansprüche 1 bis 6, ferner umfassend mindestens eine radial durchgehende Bohrung (115) unterhalb der ringförmigen, offenporigen Struktur (109) zur Fixierung von Muskelschichten der Gliedmaße.

8. Endo-Exo-Prothese (1) nach einem der Ansprüche 1 bis 7, die im Wesentlichen aus Stahl gefertigt ist, wobei alle Flächen, welche mit organischem Material direkten Kontakt haben, mit Gold oder Silber beschichtet sind.

9. Endo-Exo-Prothese (1) nach einem der Ansprüche 1 bis 8, wobei das distale Ende (105) sich außerhalb der Gliedmaße befindet und die mechanische Aufnahme (107) dazu ausgeführt ist, Funktionsteile (3) mit der Endo-Exo-Prothese (1) kraftschlüssig und/oder formschlüssig lösbar zu verbinden.

10. Endo-Exo-Prothese (1) nach Anspruch 9, wobei das Funktionsteil (3) kugelförmig (3a) oder als stilisierter Fuß (3b) ausgebildet ist.

11. Verfahren zum Anbringen einer Endo-Exo-Prothese (1) nach einem der Ansprüche 1 bis 10 an einer Gliedmaße, umfassend die Schritte
a) Absetzen eines Knochens (K) einer Gliedmaße,
b) Anbringen der Endo-Exo-Prothese (1), umfassend die Unterschritte
c) Einsetzen des proximalen Endes (103) der Endo-Exo-Prothese (1) in den Knochen (K), insbesondere in die Knochenmarkhöhle, optional bis zum direkten Kontakt mit der ringförmigen, offenporigen Struktur (109),
d) optional Festschrauben der zumindest einen Lasche (111) am Knochen (K),
e) optional Umbiegen des radial angeordneten scheibenförmigen Elements (113) um das distale Ende des Knochens (K) und optional Festschrauben am Knochen (K),
f) optional Präparation von Muskeln der Gliedmaße und Befestigung der Muskelstränge in der mindestens einen radial durchgehenden Bohrung (115).
